(19) Europäisches Patentamt
European Patent Office
Office européen des brevets

(11) Veröffentlichungsnummer : **0 069 882**
**B1**

(12) **EUROPÄISCHE PATENTSCHRIFT**

(45) Veröffentlichungstag der Patentschrift : **13.06.84**

(21) Anmeldenummer : **82105414.5**

(22) Anmeldetag : **21.06.82**

(51) Int. Cl.³ : **C 07 C103/52**, C 07 H 19/08, C 12 P 21/02, A 61 K 37/02 // (C12P21/02, C12R1/465)

(54) **Neue antibiotisch wirksame Verbindungen, Verfahren zu ihrer Herstellung sowie ihre Verwendung als Arzneimittel.**

(30) Priorität : **03.07.81 DE 3126389**

(43) Veröffentlichungstag der Anmeldung : **19.01.83 Patentblatt 83/03**

(45) Bekanntmachung des Hinweises auf die Patenterteilung : **13.06.84 Patentblatt 84/24**

(84) Benannte Vertragsstaaten : **AT BE CH DE FR GB IT LI NL SE**

(56) Entgegenhaltungen :
**EP-A- 0 057 349**
**EP-A- 0 057 812**

(73) Patentinhaber : **BAYER AG**
**Konzernverwaltung RP Patentabteilung**
**D-5090 Leverkusen 1 Bayerwerk (DE)**

(72) Erfinder : **Benz, Günter, Dr.**
**Am Bölkumer Busch 5**
**D-5620 Velbert 15 (DE)**
Erfinder : **Metzger, Karl Georg, Dr.**
**Pahlkestrasse 15**
**D-5600 Wuppertal 1 (DE)**
Erfinder : **Pfitzner, Jörg, Dr.**
**Claudiusweg 3**
**D-5600 Wuppertal 1 (DE)**
Erfinder : **Schmidt, Delf, Dr.**
**Am Eckbusch 55b**
**D-5600 Wuppertal 1 (DE)**
Erfinder : **Zeiler, Hans-Joachim, Dr.**
**Elsbeekerstrasse 46**
**D-5620 Velbert 15 (DE)**

**EP 0 069 882 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft neue antibiotisch wirksame Verbindungen, ein semisynthetisches Verfahren zu ihrer Herstellung sowie ihre Verwendung als Arzneimittel, insbesondere als antimikrobielles Mittel in der Human- und Tiermedizin.

Es ist bereits bekannt geworden, daß eine Reihe von Verbindungen mikrobieller Herkunft antimikrobielle Wirkungen besitzen. Diese Antibiotika sind teilweise in ihren Wirkungsspektren nicht voll befriedigend. Sie weisen häufig noch weitere Nachteile auf. β-Lactam-antibiotika werden oft durch Penicillinase inaktiviert, Chloramphenicol, Tetracycline und Streptomycin zeigen in vielen Fällen erhebliche unerwünschte Nebenwirkungen (vgl. Walter, Heilmeyer, Antibiotika Fibel, Georg Thieme Verlag, Stuttgart, 3. Auflage, 1969, Seiten 248, 278-280 und 311-319).

Die vorliegende Erfindung stellt Verbindungen der allgemeinen Formel (I)

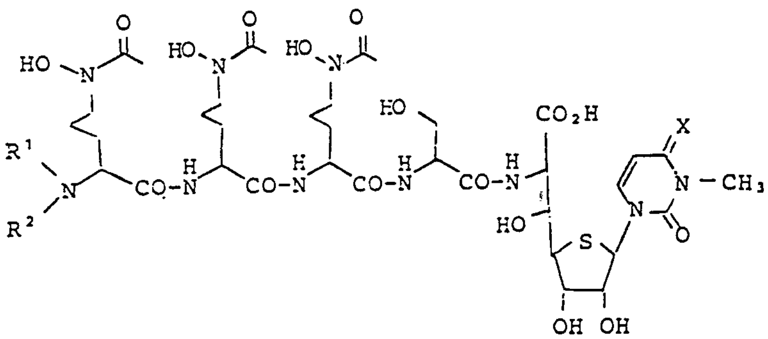

(I)

zur Verfügung.

In der Formel (I) bedeuten

X O oder N—CO—$NH_2$ und

$R^1$ eine gegebenenfalls substituierte Alkylgruppe, oder eine Acylgruppe —CO—$R_3$

$R^2$ $R^1$ oder H und

$R^3$ eine gegebenenfalls substituierte Alkyl-, Aryl- oder Aminogruppe.

Als Substituenten für $R^1$, $R^2$ und $R^3$ in der Bedeutung Alkyl kommen insbesondere Halogen, vorzugsweise Chlor; Aryl, vorzugsweise Phenyl; Heteroalkyl, vorzugsweise 5- oder 6-ringige Heteroarylreste mit vorzugsweise N, O und/oder S im Ring; und die Gruppen $COOR^4$, $OR^4$, $SR^4$ und $NR_2{}^4$ in Frage, wobei $R^4$ Wasserstoff und/oder gegebenenfalls substituiertes Alkyl oder Aryl bedeuten.

Als Substituenten für $R^4$ = Alkyl kommen die für $R^1$-$R^3$ obengenannten in Betracht.

Substituenten für Aryl, unter das erfindungsgemäß auch Heteroaryl fällt, sind die für Alkyl genannten.

Alkyl bedeutet im Zusammenhang mit vorliegender Erfindung bevorzugt offenkettiges geradliniges oder verzweigtes Alkyl mit bis zu 6 C-Atomen oder cyclisches Alkyl mit 3 bis 7 C-Atomen.

Aryl bzw. Heteroaryl steht vorzugsweise für Phenyl, Pyridyl oder Furanyl.

Wenn die genannten Reste substituiert sind, so sind 1 bis 3 Substituenten bevorzugt.

Bevorzugt sind Verbindungen mit X = O oder N—CO—$NH_2$, in denen $R_2$ = H und $R_1$ der Acylrest einer Aminosäure oder eines Di- bis Pentapeptids ist.

Besonders bevorzugt sind Verbindungen in denen X die Bedeutung N—CO—$NH_2$ und $R^1$ und $R^2$ die folgenden Bedeutungen haben

| $R^1$ | $R^2$ | |
|---|---|---|
| $CH_3$ | $CH_3$ | |
| 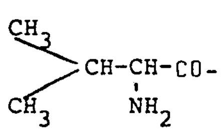 | H | 2) |
| $C_2H_5$ | $C_2H_5$ | |

(Fortsetzung)

| $R^1$ | $R^2$ | |
|---|---|---|
| $n-C_3H_7$ | $n-C_3H_7$ | |
| $n-C_4H_9$ | $n-C_4H_9$ | |
| $CH_3-CO-$ | H | |
| $C_2H_5-CO-$ | H | |
| $C_3H_7-CO-$ | H | |
| $CH_3-O-CH_2-CO-$ | H | 4) |
| 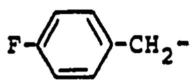 | H | |
| $NH_2-CO-$ | H | |
| 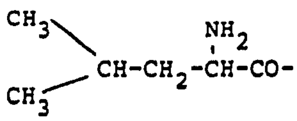 | H | |
| $HO_2C-CH_2-CH_2-CO-$ | H | |
| 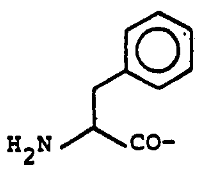 | H | |
| 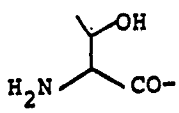 | H | |
| 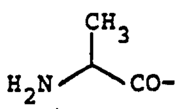 | H | 1) |
| 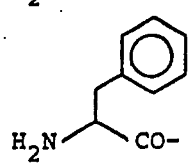 D | H | |
| $n-C_4H_9-CO-$ | H | |
| 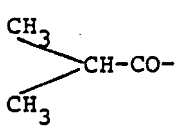 | H | |
| 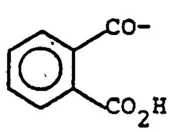 | H | |

(Fortsetzung)

| $R^1$ | $R^2$ | |
|---|---|---|
| 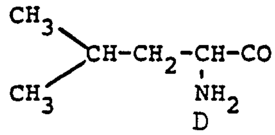 | H | |
| 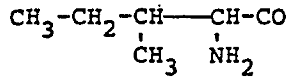 | H | |
| 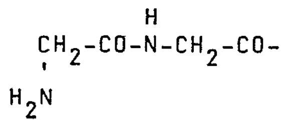 | H | 3) |

sowie Verbindungen in denen X = O ist und $R^1$ und $R^2$ folgende Bedeutung haben :

| $R^1$ | $R^2$ |
|---|---|
| 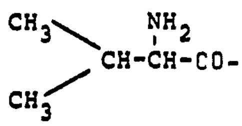 | H |
| $CH_3$ | $CH_3$ |
| 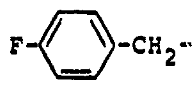 | H |
| $n-C_3H_7$ | $n-C_3H_7$ |
| 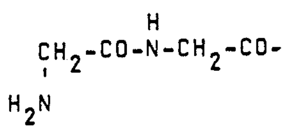 | H |

1), 2), 3), 4) bezeichnen diejenigen Verbindungen, die in Tabelle 9 mit demselben Index erscheinen.

Die neuen Verbindungen weisen starke antimikrobielle Wirkung auf.

Die erfindungsgemäßen Verbindungen können wie folgt hergestellt werden :

Zunächst wird durch submerse Kultur eines Streptomyceten-Stammes in geeigneten Nährlösungen unter geeigneten physikalischen Bedingungen ein Gemisch von Verbindungen in denen $R^1$ und $R^2$ Wasserstoff bedeuten, erzeugt. Das Gemisch wird aus der Kulturlösung durch Extraktion oder durch Absorption abgetrennt und durch weitere geeignete Methoden angereichert.

Für das Herstellungsverfahren können der Stamm Streptomyces spec. WS 116 aus der Ordnung der Actinomycetales, Familie Streptomycetaceae, Gattung Streptomyces oder von diesem abstammende Varianten und Mutanten eingesetzt werden. Dieser Stamm wurde aus einer marinen Bodensedimentprobe der Ibero-Canarischen See isoliert. Es wurde unter der Nummer DSM 1692 am 7.12.1979 bei der Deutschen Sammlung für Mikroorganismen, Göttingen hinterlegt.

a) Die Sporen sind ellipsoid. Sie haben die Größe von 0,4 – 0,7 × 0,7 – 1,2 µm und eine glatte Oberfläche.

b) Die Farbe des Luftmycels ist zu Anfang kreideweiß, im ausgereiften Zustand gelblich (Griseus-Typ).

c) Die Sporenketten sind gerade oder gewellt (Rectus-Flexibilis-Typ) und monopodial verzweigt.

d) Auf Pepton-Eisen-Agar sowie auf Tyrosin-Agar wurde kein schwarzbraunes Pigment gebildet. Der Stamm ist nicht chromogen.

Die zusammengefaßten Bestimmungsmerkmale identifizieren den Stamm WS-116 als zur Art Streptomyces griseus Waksman et Henrici gehörend.

Für das Verfahren zur Herstellung des Gemischs der Verbindungen verwendet man Nährmedien, die die üblichen Kohlenstoff- und Stickstoffquellen und die notwendigen Salze enthalten. Als Kohlenstoffquelle können verwendet werden: Kohlenhydrate, insbesondere Polysaccharide, wie Stärke oder Dextrine, Disaccharide wie Maltose oder Rohrzucker, Monosaccharide, wie Glucose oder Xylose, Zuckeralkohole, wie Mannit oder Glycerin, Carbonsäuren, wie Citronensäure, Äpfelsäure, Essigsäure oder deren Gemische, außerdem auch natürlich vorkommende Gemische, wie Malzextrakt. Überraschenderweise wurden mit Carbonsäuren, insbesondere mit Citronensäure als Haupt-C-Quelle die höchsten Wirkstoffausbeuten erzielt. Als Stickstoffquelle können die üblichen Stickstoffquellen Verwendung finden, so z. B. Eiweißstoffe, Eiweißhydrolysate, Aminosäuren wie Glutaminsäure, Asparaginsäure, Arginin, Lysin, Ornithin oder Serin, außerdem Nucleosidbasen wie Cytosin oder Uracil, Ammonium-Salze, Nitrate, natürlich vorkommende komplexe Stoffe, wie Peptone, Maisquell-wasser, Sojabohnenmehl, Fleischextrakte, Hefeextrakte und geeignete Mischungen derselben. Besonders hohe Wirkstoffausbeuten erhält man, wenn zu den üblichen komplexen N-Quellen L-Ornithin und L-Serin im Verhältnis 3 : 1, z. B. 0,3 % und 0,1 %, in sterilfiltrierter Form dem Medium zugesetzt werden.

Als Hilfsstoffe werden im Nährmedium Mineralsalze benötigt, z. B. Phosphate, Sulfate, Carbonate, Nitrate oder Chloride des Natriums, Kaliums, Calciums, Magnesiums, Eisens, Zinks, Kupfers, Molybdäns, Kobalts, Nickels und Mangans. Als bedeutsam erwies sich die Anwesenheit von etwa 0,01 % $FeCl_3$. Zum Teil sind die Mineralsalze, auch das $FeCl_3$, in den notwendigen Konzentrationen in den oben genannten Kohlenstoffoder Stickstoffquellen oder im verwendeten Wasser als Bestandteile enthalten.

Weiterhin können als Hilfsstoffe noch Antischaummittel der verschiedensten Art Verwendung finden, wie z. B. Sojaöl, Polyole oder Silikone.

Als wichtigstes Verdünnungsmittel für die Nährmedien ist Wasser zu nennen.

Bei der Durchführung des Herstellungsverfahrens wird unter aeroben bzw. mikroaerophilen Bedingungen gearbeitet; die Kultur kann gemäß üblicher Methoden, so z. B. unter Verwendung von Schüttelkulturen oder von belüfteten Fermenterkulturen im üblichen Batch- oder Fedbatch-Verfahren, durchgeführt werden. Die Prozentverhältnisse (jeweils Gewichtsprozente) der Nährlösungsbestandteile können in weiten Bereichen schwanken, im allgemeinen machen die Kohlenstoffquellen insgesamt 0,5-8 %, vorzugsweise 0,6-6 % aus, die Stickstoffquellen insgesamt 0,05 bis 4 %, vorzugsweise 0,5 bis 2 % aus; die Salze liegen in üblichen Konzentrationen vor, vorzugsweise im Bereich zwischen 0,001 bis 0,5 Gew.-%. Die Antischaummittel liegen in 0- bis 1-%iger Konzentration vor. Die zur Sterilisation angewandten Temperaturen liegen bei 100 bis 140 °C, vorzugsweise bei 120 bis 130 °C, empfindliche Substanzen wie Aminosäuren werden sterilfiltriert.

Die pH-Werte der wachsenden Kulturen liegen zwischen 5 und 10, vorzugsweise zwischen 6 und 9,5. Die Züchtungstemperatur kann zwischen 15 und 35 °C, vorzugsweise zwischen 20 und 30 °C liegen. Es hat sich herausgestellt, daß die Menge des sich in der Kulturbrühe anreichernden Produkte im allgemeinen ihr Maximum etwa 1 bis 10, vorzugsweise etwa 2 bis 5 Tage nach Züchtungsbeginn erreicht. Der Endpunkt der Fermentation wird mit Hilfe von biologischen Tests ermittelt. (Wirkung gegen E. coli in einem üblichen Agardiffusionstest).

Die erhaltenen Verbindungen werden aud dem Kulturfiltrat durch Extraktion mit Mischungen aus Phenol/Chloroform oder aber durch Absorption an Aktivkohle bzw. an geeignete Harze isoliert. Vorteilhaft ist die Bindung der Verbindungen an unspezifische Adsorberharze auf Polystyrolbasis (z. B. Amberlite® XAD der Fa. Rohm & Haas oder Lewatit® OC 1031 der Fa. Bayer). Es wurde gefunden, daß die Verbindungen besonders fest von derartigen Harzen gebunden wird, wenn man vor dem Adsorptionsvorgang Eisensalze insbesondere Eisenchlorid in Konzentrationen von 0,05 bis 0,2, insbesondere ca. 0,1 g/Liter Kulturbrühe hinzugibt. Die Adsorption führt man in pH Bereich 3-9, insbesondere im Bereich 5-7 durch. Die Desorption der Verbindungen wird fraktioniert durch Mischungen aus Wasser und organischen Lösungsmitteln, insbesondere Wasser/Methanol vorgenommen. Die aktiven Fraktionen werden vereinigt, auf ein kleines Volumen eingeengt und lyophilisiert. Man erhält ein 0,5-3%iges Rohprodukt, das die Verbindungen enthält.

Ausgehend von diesem Rohprodukt kann die weitere Anreicherung der Verbindungen durch eine Kombination von Anionen- (z. B. DEAE-Sephadex® A 25, Fa. Pharmacia) bzw. Kationen-Austauschchromatografie (z. B. SP- oder CM-Sephadex® C 25, Fa. Pharmacia) durchgeführt werden. Man erhält dadurch ein 30-50%iges Präparat, da begleitende Peptide nicht abgetrennt werden. Die Abtrennung dieser Peptide gelingt durch Adsorptions-bzw. Verteilungschromatografie der ca. 30-50 %iger Verbindung an Kieselgel im System Isobutanol/Ethanol/25 % konz. Ammoniak = 9/1/5. (Volumenteile).

Dieses Trennverfahren ist jedoch mit Substanzverlusten verbunden.

Wesentlich einfacher gelingt die Trennung durch Affinitätschromatografie in einer $Fe^{+++}$-haltigen Säule. Dazu überführt man einen Kationenaustauscher auf Polystyrolharz oder Acrylharzbasis, (z. B. Dowex® 50 WX 4, Fa. DOW Chemical) oder auf Polydextranbasis (z. B. Sephadex® C 25, Fa. Pharmacia) mit $FeCl_3$-Lösung in die $Fe^{+++}$ Form. Auf das Harz in der $Fe^{+++}$ Form wird nun die Lösung des Rohproduktes aufgetragen und anschließend mit Wasser nachgewaschen. Dann eluiert man mit einem Puffer hoher Ionenstärke, z. B. 0,2 m $NaH_2PO_4$/0,3 m NaCl. Dieser Puffer eluiert die Hauptmenge der inaktiven Begleitpeptide. Anschließend eluiert man die wirksamen Substanzen von der Säule mit dem gleichen Puffer, jedoch unter Zusatz von 0,05 m Ethylendiamintetraessigsäure oder einem anderen Eisenkomplexbildner (z. B. Citrat). Die aktiven Fraktionen werden vereinigt und über eine Säule mit unspezifischem Adsorptionsharz (z. B. Lewatit® OC 1031, Fa. Bayer) gegeben, wobei die wirksamen Substanzen gebunden werden. Man eluiert mit Methanol, engt ein und lyophilisiert.

Die Verbindungen mit $R^1$ und $R^2$ = H werden durch Verteilungschromatografie an Sephadex® G 25/n BuOH/iso BuOH/0,2 m $(NH_4)_2SO_4$ = 2/1/1 rein erhalten. Die Reindarstellung der Produkte wird ferner durch einfache Chromatografie des Wirkstoffgemisches an einer CM-Cellulose-Säule in der $H^+$-Form mit dest. Wasser ohne irgenwelche Zusätze durchgeführt. Das Eluat, in Fraktionen aufgefangen, wird lyophilisiert.

Diese beiden Verbindungen, können in eisenhaltiger Form (komplex gebunden) und in eisenfreier Form auftreten. Die eisenfreie Verbindung mit X = O kann wie folgt charakterisiert werden (vgl. EP-A-0057812) :

(1) Die Elementaranalyse C 43,8 % ; H 6,7 % ; N 14,1 % ; O 30,3 % ; S 4,1 %.

Es muß hier darauf hingeweisen werden, daß bei höhermolekularen Naturstoffen die Fehlerbreite der Elementaranalyse größer sein kann, als allgemein üblich, daher ist einer genaue Bestimmung der Summenformel oft nicht möglich (R. B. Woodward, Angew. Chem. 69, S. 50-51 (1957)).

(2) Die gefriergetrocknete Verbindung schmilzt zwischen 180 und 185 °C und zersetzt sich bei weiterer Erwärmung.

(3) Ultraviolettabsorptionsspectrum : Das UV-Spektrum wurde an einer wässrigen Lösung der Verbindung (c = 2 863 mg in 50 ml $H_2O$) aufgenommen. Die Spektren in saurer (bzw. basischer) Lösung wurden an einer Lösung gemessen, die durch Zusatz von 100 Mikrolitern 1 n Salzsäure (bzw. Natronlauge) zu 3 ml der obigen Lösung hergestellt wurde.

Tabelle 1

Maxima (λ max) und Extinktionen [E 1 %/cm] der Verbindung

| | λ max [nm] | [E 1 %/cm] |
|---|---|---|
| Neutral | 267 | 103 |
| Sauer | 267 | 97.10 |
| Basisch | — | — |

(4) Das IR-Absorptionsspektrum der Verbindung wird in Figur 1 wiedergegeben. (Abszisse : Wellenzahl in $cm^{-1}$ Ordinate : Absorption).

Es zeigt, wenn die Substanz zu KBr Preßlingen verpreßt wird, bei folgenden Wellenlägen (ausgedrückt in $cm^{-1}$) Absorptionsbanden :

Tabelle 2

Wellenlängen des IR-Absorptionsspektrums

| Wellenlänge in $cm^{-1}$ | Wellenlänge in $cm^{-1}$ |
|---|---|
| 3 384 | 1 210 |
| 2 918 | 1 160 |
| 1 654 | 1 090 |
| 1 615 | 970 |
| 1 540 | |
| 1 457 | |
| 1 415 | |
| 1 390 | |
| 1 300 | |
| 1 240 | |

(5) Das $^1$H-Kernresonanzspektrum gibt die Signallage in Teilen pro Million (ppm) und Schwingungen pro Sekunde gemäß Figur 2 an. Es wurde an einer wässrigen Lösung der Verbindung mit TMS-Na-Salz als Standard (extern) an einem WH-360 Spektrometer der Firma Bruker bei einer Feldstärke von 360 MHZ aufgenommen.

(6) Das 13-C-Kernresonanzspektrum wurde an einem WM-250 Spektrometer der Fa. Bruker bei einer Feldstärke von 62,71 MHZ an einer wässrigen Lösung der Verbindung aufgenommen, wobei auf Dioxan als externen Standard (Shiftlage 67 400 ppm rel. zu TMS = 0) umgerechnet wurde.

Das 13-C-Kernresonanzspektrum gemäß Abbildung 3 zeigt die folgenden Signale angegeben in Teilen pro Million (ppm) und Schwingungen pro Sekunde (HZ) in ihren relativen Intensitäten an :

Tabelle 3

Schiftlagen und Intensitäten der Signale im 13-C-Kernresonanzspektrum bezogen auf Dioxan = 67 400 ppm (extern)

| Signal NS | rel. Intensität | Signallage (ppm) |
|---|---|---|
| 1 | 11 061 | 20 222 |
| 2 | 2 101 | 20 511 |
| 3 | 3 960 | 22 305 |
| 4 | 4 026 | 23 173 |
| 5 | 3 867 | 23 364 |
| 6 | 16 325 | 28 919 |
| 7 | 4 636 | 47 906 |
| 8 | 6 720 | 48 097 |
| 9 | 4 263 | 51 317 |
| 10 | 4 490 | 53 464 |
| 11 | 4 625 | 54 302 |
| 12 | 5 906 | 54 45 |
| 13 | 6 302 | 56 346 |
| 14 | 4 260 | 58 317 |
| 15 | 5 171 | 61 993 |
| 16 | 4 332 | 64 581 |
| 17 | 5 260 | 70 022 |
| 18 | 5 100 | 75 962 |
| 19 | 5 436 | 80 536 |
| 20 | 5 437 | 102 328 |
| 21 | 5 790 | 142 604 |
| 22 | 4 421 | 153 765 |
| 23 | 5 004 | 166 249 |
| 24 | 3 694 | 170 322 |
| 25 | 4 663 | 171 837 |
| 26 | 3 740 | 174 028 |
| 27 | 3 837 | 174 395 |
| 28 | 5 046 | 174 778 |
| 29 | 2 662 | 174 925 |
| 30 | 5 686 | 175 792 |

(7) Der optische Drehwert beträgt $[\alpha]_D^{20} = -20{,}216$ (C = 0,395 1 % in Wasser).

(8) Die Verbindung ist unbeschränkt in Wasser löslich, löst sich etwas in Methanol, DMF und DMSO und ist schwerlöslich in Chloroform, Äher, Essigester und Petroläther.

(9) Die Verbindung ist ein farbloser, amorpher Feststoff, dessen wässrige Lösung neutral reagiert.

(10) Die $R_f$-Werte der Verbindung in der eisenfreien und eisenhaltigen Form im Vergleich mit anderen Verbindungen in verschiedenen Laufmitteln gibt Tabelle 4.

a) DC-Fertigplatten Kieselgel 60 F 254 (Merck)
Anfärbung : 1. Ninhydrin ; 2. 5 % $FeCl_3 \times 6\ H_2O$ in 0,5 n HCl
Fließmittel 1 (FM 1) : Isobutanol/Ethanol/Ammoniak = 9/1/5
Fließmittel 2 (FM 2) : Isobutanol/Ethanol/Ammoniak = 4/1/5
10 cm Laufstrecke/Auftrag 50 µg in dest. Wasser.

Tabelle 4a

| Substanz | FM 1 | FM 2 |
|---|---|---|
| Neomycinsulfat | 0,01 | 0,16 |
| 2-Desoxystreptamin × 2 HCl | 0,03 | 0,19 |
| Sisomicin Base | 0,14 | 0,42 |
| Verbindung mit $R^1/R^2$ = H, X = 0 | | |
| eisenfrei | 0,02 | 0,26 |
| dto, einsenhaltig | 0 | 0,23 |

b) DC-Fertigplatten Cellulose F (Merck)
Anfärbung : 1. Ninhydrin, 2.5 % $FeCl_3 \times 6\ H_2O$ in 0,5 n HCl
Fließmittel 1 (FM 1) : 1-Butanol/Eisessig/dest. Wasser 4/1/5
Fließmittel 2 (FM 2) : 1-Butanol/Eisessig/dest. Wasser 4/1/2
Fließmittel 3 (FM 3) : 1-Propanol/Pyridin/Eisessig/dest. Wasser 15/10/3/12
10 cm Laufstrecke/Auftrag 50 µg in dest. Wasser.

Tabelle 4b

| Substanz | FM 1 | FM 2 | FM 3 |
|---|---|---|---|
| Neomycinsulfat | 0,06 | 0,06 | 0,06 |
| 2-Desoxystreptamine × 2 $H_2O$ | 0,10 | 0,13 | 0,42 |
| Sisomicin Base | 0,16 | 0,27 | 0,49 |
| Verbindung mit $R^1/R^2$ = H, X = 0 | | | |
| eisenfrei | 0,37 | 0,42 | 0,83 |
| dto, eisenhaltig | 0,14 | 0,25 | 0,74 |

(11) Die Verbindung läßt sich in seiner eisenfreien Form auf der Dünnschichplatte mit $FeCl_3$ +), alk. Kaliumpermanganat, Jod, Ninhydrin und im UV-Licht bei 254 oder 280 nm durch Fluorescenzlöschung sichtbar machen.

Die Verbindung mit X = N—CO—$NH_2$ kann als eisenfreie Form wie folgt charakterisiert werden (vgl. EP-A-0057349) :

(1) Die Elementaranalyse C 42,3 % ; H 6,2 % ; N 15,7 % ; O 31,4 % ; S 3,6 %.

(2) Die gefriergetrocknete Verbindung zersetzt sich bei etwa 185 °C.

(3) Ultraviolettabsorptionsspectrum : Das UV-Spektrum wurde an einer wäßrigen Lösung der Verbindung (c = 1 786 mg in 25 ml $H_2O$) aufgenommen. Die Spektren in saurer (bzw. basischer Lösung) wurden an einer Lösung gemessen, die durch Zusatz von 100 Mikrolitern 1 n Salzsäure (bzw. Natronlauge) zu 3 ml der obigen Lösung hergestellt wurde.

+) Die Sprühreagentien wurden nach den üblichen Vorschriften (z. B. E Stahl Dünnschichtchromatographie 2. Auflage, Springer Verlag, Berlin) hergestellt.

Tabelle 5

Maxima (λ max) und Extinktionen [E 1 %/cm] der Verbindung

| | λ max [nm] | [E 1 %/cm] |
|---|---|---|
| Neutral | 282 | 123 |
| Sauer | 304 | 108 |
| Basisch | 277 | 118 |

(4) Das IR-Absorptionsspektrum der Verbindung wird in Figur 4 wiedergegeben. (Abszisse : Wellenzahl in $cm^{-1}$, Ordinate : Absorption).

Es zeigt, wenn die Substanz zu KBr Preßlingen verpreßt wird, bei folgenden Wellenlängen (ausgedrückt in $cm^{-1}$) Absorptionsbanden :

Tabelle 6

| Wellenlänge in $cm^{-1}$ | Wellenlänge in $cm^{-1}$ |
|---|---|
| 3 388 | 1 070 |
| 2 944 | 1 050 |
| 1 695 | 975 |
| 1 648 | |
| 1 545 | |
| 1 457 | |
| 1 417 | |

Tabelle 6 (Fortsetzung)

| Wellenlänge in $cm^{-1}$ | Wellenlänge in $cm^{-1}$ |
|---|---|
| 1 390 | |
| 1 299 | |
| 1 240 | |

(5) Das $^{1}H$-Kernresonanzspektrum gibt die Signallage in Teilen pro Million (ppm) und Schwingungen pro Sekunde gemäß Figur 5 an. Es wurde an einer wäßrigen Lösung der Verbindung mit TMS-Na-Salz als Standard (extern) an einem WH-360 Spektrometer der Firma Bruker bei einer Feldstärke von 360 MHZ aufgenommen.

(6) Das 13-C-Kernresonanzspektrum wurde an einem WM-250 Spektrometer der Fa. Bruker bei einer Feldstärke von 62.71 MHZ an einer wäßrigen Lösung der Verbindung aufgenommen, wobei auf Dioxan als externen Standard (Shiftlage 67 400 ppm rel. zu TMS = 0) umgerechnet wurde.

Das 13-C-Kernresonanzspektrum gemäß Figur 6 zeigt die folgende Signale angegeben in Teilen pro Million (ppm) und Schwingungen pro Sekunde (HZ) in ihren relativen Intensitäten an :

Tabelle 7

Shiftlage und Intensitäten der Signale im 13-C-Kernresonanzspektrum bezogen auf Dioxan = 67 400 ppm (extern)

| Signals NS | rel. Intensität | Signallage (ppm) |
|---|---|---|
| 1 | 3 344 | 175 878 |
| 2 | 2 094 | 174 610 |
| 3 | 1 628 | 174 289 |
| 4 | 1 634 | 174 032 |
| 5 | 1 708 | 171 786 |
| 6 | 1 25 | 167 843 |
| 7 | 1 966 | 156 220 |
| 8 | 2 580 | 153 171 |
| 9 | 3 806 | 138 745 |
| 10 | 3 979 | 98 082 |
| 11 | 3 729 | 80 173 |
| 12 | 3 207 | 76 001 |
| 13 | 3 044 | 69 984 |
| 14 | 83 487 | 67 400 |
| 15 | 2 996 | 64 094 |
| 16 | 4 082 | 62 072 |
| 17 | 1 751 | 58 606 |
| 18 | 2 995 | 56 392 |
| 19 | 4 129 | 54 418 |
| 20 | 4 430 | 54 322 |
| 21 | 2 449 | 54 017 |
| 22 | 2 983 | 51 064 |
| 23 | 3 998 | 48 111 |
| 24 | 2 442 | 29 834 |
| 25 | 4 590 | 29 048 |
| 26 | 5 047 | 28 967 |
| 27 | 2 559 | 23 319 |
| 28 | 2 276 | 23 223 |
| 29 | 1 510 | 22 634 |
| 30 | 7 743 | 20 222 |

(7) Der optische Drehwert beträgt sofort nach dem Lösen $[\alpha]_D^{20} = -27{,}725°$ (c = 0,272 3 % in Wasser).

(8) Die Verbindung ist unbeschränkt in Wasser löslich, löst sich etwas in Methanol, DMF und DMSO und ist schwerlöslich in Chloroform, Ether, Essigester und Petrolether.

(9) Die Verbindung ist ein farbloser, amorpher Feststoff, dessen wäßrige Lösung neutral reagiert.

(10) Die $R_f$-Werte der Verbindung in der eisenfreien und eisenhaltigen Form im Vergleich mit anderen Verbindungen in verschiedenen Laufmitteln gibt Tabelle 8.

a) DC-Fertigplatten Kieselgel 60 F 254 (Merck)
Anfärbung : 1. Ninhydrin ; 2. 5 % $FeCl_3 \times 6\ H_2O$ in 0,5 n HCl
Fließmittel (FM 1) : Isobutanol/Ethanol/Ammoniak = 9/1/5 (Volumenteile)
Fließmittel 2 (FM 2) : Isobutanol/Ethanol/Ammoniak = 4/1/5 (Volumenteile)
10 cm Laufstrecke/Auftrag 50 µg in dest. Wasser

Tabelle 8a

| Substanz | FM 1 | FM 2 |
|---|---|---|
| Neomycinsulfat | 0,01 | 0,16 |
| 2-Desoxystreptamin × 2 HCl | 0,03 | 0,19 |
| Sisomicin Base | 0,14 | 0,42 |
| Verbindung $R^1/R^2$ = H, X = N—CO—$NH_2$, | | |
| eisenfrei | 0,02 | 0,25 |
| dto, eisenhaltig | 0 | 0,22 |

b) DX-Fertigplatten Cellulose F (Merck)
Anfärbung : 1. Ninhydrin ; 2. 5 % $FeCl_3 \times 6$ HCl
Fließmittel 1 (Fm 1) : 1-Butanol/Eisessig/dest.
Wasser 4/1/5 Fließmittel 2 (Fm 2) : 1-Butanol/Eisessig-dest. Wasser 4/1/2 Fließmittel 3 (FM 3) : 1-Propanol/Pyridin/Eisessig/dest. Wasser 15/10/3/12
10 cm Laufstrecke/Auftrag 50 µg in dest. Wasser

Tabelle 8b

| Substanz | FM 1 | FM 2 | FM 3 |
|---|---|---|---|
| Neomycinsulfat | 0,06 | 0,06 | 0,06 |
| 2-Desoxystreptamine × 2 $H_2O$ | 0,10 | 0,13 | 0,42 |
| Sisomicin Base | 0,16 | 0,27 | 0,49 |
| Verbindung $R^1/R^2$ = H, X = N—CO—$NH_2$ | | | |
| eisenfrei | 0,27 | 0,33 | 0,80 |
| dto, eisenhaltig | 0,15 | 0,19 | 0,71 |

(11) Die Verbindung läßt sich in ihrer eisenfreien Form auf der Dünnschichtplatte mit $FeCl_3$+), alk. Kaliumpermanganat, Jod, Ninhydrin und im UV-Licht bei 254 oder 280 nm durch Fluorescenzlöschung sichtbar machen.

Diese Verbindungen in eisenfreier Form, werden dann an der endständigen Aminogruppe des Ornithins derivatisiert.

Durch reduktive Alkylierungen mit aliphatischen und aromatischen Aldehyden und Natriumcyanoborhydrid im wäßrigen schwach sauren Medium können die entsprechenden Mono- und Dialkylverbindungen erhalten werden.

Die Acylierungen können unter modifizierten Schotten-Baumann-Bedingungen im neutralen wäßrigalkoholischen Medium mit Carbonsäureanhydriden erfolgen.

Die Aminoacylierungen können mit o-Nitrophenylsulfenyl (NPS) oder t-Butoxycarbonyl (BOC) geschützten L- und D-Aminosäuren oder Peptiden nach Aktivierung über die gemischten Anhydride oder die aktiven Ester erfolgen.

Die Komponenten A und B (Definition vgl. Beispiel e)) werden bei allen Umsetzungen ungeschützt eingesetzt.

Die Reinigung kann durch Chromatographie an Celluloseionenaustauscher und an Kieselgel erfolgen.

Die gute antimikrobielle Wirksamkeit der erfindungsgemäßen Verbindungen wird durch in vitro-Versuche demonstriert.

In vitro wird im Agar-Verdünnungstest nach dem international üblichen Test (American National Committee for Clinical Laboratory Standards = NCCLS) Wirksamkeit gegen nachfolgend aufgeführte Keime gefunden.

+) Die Sprühreagentien wurden nach den üblichen Vorschriften (z. B. E. Stahl Dünnschichtchromatographie 2. Auflage, Springer Verlag, Berlin) hergestellt.

Tabelle 9

| Verbindung * | Keim | MHK µg/ml |
|---|---|---|
| 1) | E. coli Neumann | ≤ 0,06 |
| | Staph. aureus 1756 | ≤ 0,06 |
| 2) | E. coli Neumann | ≤ 0,01 |
| | Staphylococcus aureus 133 | ≤ 0,01 |
| 3) | E. coli Neumann | ≤ 0,06 |
| | Klebsiella 57 US | ≤ 0,06 |
| 4) | E. coli Neumann | ≤ 0,06 |

* s. Indizierung S. 3 ff.

Die Bestimmung der minimalen Hemmkonzentration (MHK) erfolgte mit einem Inoculator im Agarverdünnungsverfahren mit einer Einsaatdichte von $10^4$ pro Impf-Punkt. Die MHK ist die Konzentration, bei der keine Bakterienkolonien wachsen.

Die erfindungsgemäßen Wirkstoffe sind gegen ein breites Spektrum von Mikroorganismen wirksam. Mit ihrer Hilfe können gramnegative und grampositive Bakterien und bakterienähnliche Mikroorganismen bekämpft sowie die durch diese Erreger hervorgerufenen Erkrankungen verhindert, gebessert und/oder geheilt werden.

Besonders wirksam sind die erfindungsgemäßen Wirkstoffe gegen Bakterien und bakterienähnliche Mikroorgansimen. Sie sind daher besonders gut zur Prophylaxe und Chemotherapie von lokalen und systematischen Infektionen in der Human- und Tiermedizin geeignet, die durch diese Erreger hervorgerufen werden.

Beispielsweise können lokale und/oder systematische Erkrankungen behandelt und/oder verhindert werden, die durch die folgenden Erreger oder durch Mischungen der folgenden Erreger verursacht werden :

Micrococcaceae, wie Staphylokokken, z. B. Staphylococcus aureeus (Staph. = Staphylococcus) ;

Enterobacteriaceae, Escherichia-Bakterien, z. B. Escherichia coli, Enterobacter-Bakterien, z. B. E. aerogenes, E. cloacae, Klebsiella-Bakterien, z. B. K-pneumoniae, K. pneumoniae, K. ozaenae, Erwiniae, z. B. Erwinia spec., Serratia, z. B. Serratia marcescenc (E. = Enterobacter) (K. = Klebsiella).

Pseudomonadaceae, wie Pseudomonas-Bakterien, z. B. Pseudomonas aeruginosa.

Die erfindungsgemäßen Verbindungen können im üblichen pharmazeutischen Zusammensetzungen angewendet werden. Die pharmazeutischen Zubereitungen enthalten mindestens einen der erfindungsgemäßen Wirkstoffe und übliche, geeignete, nicht-toxische Träger- und Hilfsstoffe.

Geeignete Zubereitungen und die dazu notwendigen Hilfs- und Trägerstoffe sind beispielsweise aus der Deutschen Offenlegungsschrift 25 10 161 bekannt.

## Beispiele

### Herstellung der Ausgangsverbindungen mit $R^1 = R^2 = H$

### Beispiel a)

Die Nährlösung in der der Produktionsstamm Streptomyces spec. WS 116 in den Vorkulturen kultiviert wird, setzt sich aus 2 Gew.-% Glucose, 1,3 % Hefeextrakt, 0,05 % Polyol und Leitungswasser zusammen. Der pH wird vor dem Sterilisieren auf 6,5 eingestellt. 4 × 1 000 ml-Erlenmeyerkolben, die je 150 ml dieser Nährlösung enthalten, werden mit dem Produktionsstamm beimpft und 4 Tage bei 28 °C auf einer Rundschüttelmaschine bei 220 Umdrehungen/min inkubiert. Mit diesen Vorkulturen wird eine zweite Vorkultur in einem-Labor-Fermenter, der 20 l Nährlösung enthält, beimpft und bei 200 Umdrehungen/min, 10 l Luft/min und 25 °C 3 Tage inkubiert. Mit 10 l dieser Kultur wird ein Produktionsfermenter beimpft, der 600 l Nährlösung mit folgender Zusammensetzung enthält : 0,7 Gew.-% Citronensäure, 0,8 % Hefeextrakt, 0,2 % entfettetes Sojabohnenmehl, 0,2 % Maisquellwasser und 0,05 % Silicon in Leitungswasser. Der pH dieser Nährlösung wird mit Kalilauge auf 6,4 vor der Sterilisation eingestellt. Die Inkubation der Produktionskultur erfolgt über 2-4 Tage bei 25 °C bei einer Rührung von 100 Umdrehungen/min und einer Belüftung von nur 50 l Luft/min. Die Fermentation wird bei optimaler antibiotischer Hemmaktivität der Kultur abgestoppt.

### Beispiel b)

2 × 150 ml Vorkultur werden im Beispiel a) angegeben, angezogen. Mit diesen Vorkulturen wird ein

10 l-Produktionsfermenter beimpft, dessen Nährlösung, in Leitungswasser angesetzt, folgende Zusammensetzung enthält :

0,7 Gew.-% Citronensäure,
0,8 Gew.-% Hefeextrakt,
0,2 Gew.-% entfettetes Sojabohnenmehl,
0,2 Gew.-% Maisquellwasser,
0,3 Gew.-% L-Ornithin,
0,1 Gew.-% L-Serin und
0,05 Gew.-% Silicon.

Alle Bestandteile außer Ornithin und Serin werden wie üblich im Kulturgefäß sterilisiert. Es wird ein pH-Wert von 6,4 vor der Sterilisation eingestellt. Ornithin und Serin werden in $H_2O$ dest. gelöst dem Ansatz sterilfiltriert zugesetzt.

Die Inkubation der Produktionskultur erfolgt über 2-4 Tage bei 25° mit einer Rührung von 200 Umdrehungen/min und einer Belüftung von nur 1,5 l Luft/min. Die Fermentation wird bei optimaler antibiotischer Hemmaktivität des Kulturüberstandes abgestoppt.

Beispiel c)

4 000 l Kulturbrühe (pH = 9,06) wurden mit 50 l 1 : 1 verdünnter HCl auf pH 6,2 eingestellt. Man setzte 400 g $FeCl_3 \cdot 6\ H_2O$ hinzu, rührte und gab dann 25 l verdünnte NaOH bis pH 7 unter Rühren hinzu. Dann wurde mit 200-250 l/h im Westfalia-Separator separiert. Der Überstand wurde durch eine 30 × 70 cm hoch mit Lewatit® OC 1031 (= unsp. Adsorptionsharz der BAYER AG) gefüllte Säule gegeben, der Durchlauf kanalisiert da inaktiv. Die Säule wurde mit 1 000 l entionisiertem Wasser gewaschen, das Waschwasser war inaktiv und wurde verworfen. Die Säule wurde nun mit 1 000 l 15 % Methanol gewaschen, dieses inaktive Waschwasser wurde ebenfalls verworfen. Man eluierte die Aktivität nun mit 50 % Methanol von der Säule und sammelte 100 l Franktionen. Die aktiven Eluate 2 und 3 wurden vereinigt, eingeengt im Dünnschichtverdampfer auf ca. 20 l und dann lyophilisiert. Man erhielt 342 g Rohprodukt mit einem Gehalt von ca. 2,5 %.

Beispiel d)

Man löste die obige Rohsubstanz in 6 Litern $H_2O$ und versetzte unter Rühren mit 25 ml 50 %iger $FeCl_3$-Lösung. Der sich bildende Niederschlag wurde nach 15 Minuten Rühren abzentrifugiert (Hettich Rota Magna Zentrifuge, 1,5 l Becher, 30 min, 4 000 Upm). Der Überstand wurde mit Schwerkraft über eine 8 × 45 cm hoch mit SP-Sephadex® C 25 $Fe^{+++}$ gefüllte Säule gegeben. Die Fließrate betrug 4 l pro Stunde. Man wusch die schwarz gefärbte Säule mit 5 l dest. $H_2O$ nach und schloß dann 10 l 0,2 m $NaH_2PO_4$/0,3 NaCl Puffer an. Durchlauf und Waschwasser enthielten weniger als 5 % der eingesetzten antibiotischen Aktivität. Die jetzt nur noch hellbraun gefärbte Säule wurde nun mit 0,2 m $NaH_2PO_4$/0,3 m NaCl/0,05 m AeDTA eluiert (Fließrate 2-3 l/h), das Säuleneluat fraktioniert in 500 ml Portionen aufgefangen. Die aktiven Fraktionen 6-14 wurden vereinigt und über eine 5 × 40 mit Lewatit® OC 1031 gefüllte Säule gegeben. Die Fließrate betrug 3 l/h. Man wusch anschließend mit destilliertem Wasser bis mit $AgNO_3$ keine $Cl^-$ im Säuleneluat nachzuweisen war (ca. 6 Liter, Fließrate 5 l/h). Anschließend eluierte man die Säule mit 3 Liter 90 % Methanol, die im batch aufgefangen, eingeengt und lyophilisiert wurden. Ausbeute : 6,74 g, 82,6 (beide Komponenten).

Beispiel e)

Die halbe Ausbeute aus Beispiel c) = 3,37 g wurden in 100 ml dest. $H_2O$ gelöst. Die Leitfähigkeit betrug 210 µS.

Diese Lösung wurde auf eine 5 × 30 cm mit CM-Cellulose in der $H^+$-Form (CM-Cellulose C 52, Fa. Whatmann) gefüllte Säule aufgetragen. Man entwickelte mit dest. Wasser bei einer Fließrate von 840 ml/h. Das Eluat wurde an Hand der Refraktions-, Leitfähigkeits und Extraktionskurve geschnitten. Man erhielt Vorlauf (inaktiv) 980 mg

| | |
|---|---|
| Fraktion 1 | 427 mg |
| Fraktion 2 | 674 mg |
| Fraktion 3 | 363 mg |
| Fraktion 4-6 | 475 mg |
| Fraktion 7 | 253 mg |
| Fraktion 8 | 96 mg |

Fraktion 1 ist die Verbindung mit X = O (im folgenden Komponente A genannt).

Die Fraktionen 2-8 enthalten die Verbindung mit X = N—CO—$NH_2$ (im folgenden Komponente B genannt).

## Derivatisierung

### Beispiel 1

N-Dimethylamino-Derivat der Komponente B

100 mg (0,1 mmol) Komponente B werden in 2 ml Wasser, 0,5 ml Acetonitril gelöst und mit 0,1 ml 37 % wäßriger Formaldehydlösung versetzt. Nach Zugabe eines Tropfen Eisessig werden portionsweise bei 25 °C 19 mg (0,3 mmol) Natriumcyanoborhydrid zugegeben und 16 h gerührt. Die Lösung wird eingeengt und über eine Merck Fertigsäule Größe A (240-10) Lichropep® Si 60 getrennt. Als Laufmittel wurde ein Gradient Acetonitril : Wasser 4 : 1 → 2 : 1 verwendet. Die einheitlichen Fraktionen werden gefriergetrocknet.

Ausbeute 56,7 mg (55,5 %).

$^1$H-NMR ($D_2$ 0,250 MHz) : δ = 1.50-1.98 (m ; 12 H, β, γ-$CH_2$-Ornithin), 2.15 (s ; 9H, N-Acetyl), 2.67 (s ; 6H, —$N(CH_3)_2$), 3.34 (s ; 3H, > N—$CH_3$), 5.94 (d, J = 6 Hz ; 1H, H-1'), 6.22 (d, J = 8 Hz ; 1H, H-5), 8.20 (d, J = 8 Hz ; 1H, H-6).

UV (qualitativ, pH 1) : $\lambda_{max}$ = 304 nm.

### Beispiel 2

N-Dimethylamino-Derivat der Komponente A

50 mg (0,052 mmol) Komponente A werden in 1 ml Wasser und 0,25 ml Acetonitril gelöst und mit 0,05 ml 37 % wäßriger Formaldehydlösung versetzt. Nach Zugabe eines Tropfens Eisessig werden 9,5 mg Natriumcyanoborhydrid bei 25 °C portionsweise zugegeben und 16 h gerührt. Die Lösung wird eingeengt und an 7,5 g Kieselgel (0,063-0,2 mm) chromatographiert. Als Laufmittel wurde ein Gradient Acetonitril : Wasser 9 : 1 → 2 : 1 verwendet. Die einheitlichen Fraktionen werden gefriergetrocknet.

Ausbeute : 16 mg (31,4 %).

$^1$H-NMR ($D_2$ 0,250 MHz) : δ = 1.49-1.92 (m ; 12H, β, γ-$CH_2$-Ornithin), 2.13 (s ; 9H, N-Acetyl), 2.56 (s ; 6H, —$N(CH_3)_2$), 3.29 (s ; 3H, > N—$CH_3$), 5.94 (d, J = 6 Hz ; 1H, H-1'), 5.98 (d, J = 8 Hz ; 1H, H-5), 8.42 (d, J = 8 Hz ; 1H, H-6).

UV (qualitativ, pH 1) : $\lambda_{max}$ = 266 nm.

### Beispiel 3

N-Methocyacetyl-Derivat der Komponente B

200 mg (0,2 mmol) Komponente B werden in 10 ml wasserfreiem Methanol suspendiert. Nach Zugabe von 2 ml Methoxyessigsäureanhydrid wird der Ansatz 16 h bei 25 °C gerührt, wobei der pH mit Triethylamin auf 7 gehalten wurde. Der Ansatz wird anschließend mit 200 ml wasserfreiem Diethylether versetzt. Die etherische Lösung wird verworfen, das ausgefallene Produkt wird 30 min. mit 10 ml 0,5 n Ammoniak/Methanol gerührt und einrotiert. Der Rückstand wird an 20 g Kieselgel (0,063-0,2 mm) chromatographiert. Als Laufmittel wurde ein Gradient Acetonitril ; Wasser 9 : 1 → 8 : 2 verwendet.

Ausbeute : 115,8 mg (54,4 %).

$^1$H-NMR ($D_2$0,250 MHz) : δ = 1,56-1,92 (m ; 12H, β, γ-$CH_2$-Ornithin), 2.14 (s ; 9H, N-Acetyl), 3.33 (s ; 3H, > N—$CH_3$), 3.43 (s ; 3H, O—$CH_3$), 4.05 (s ; 2H, —$OCH_2$—CO), 5.94 (d, J = 6 ; 1H, H-1'), 6.22 (d, J = 8 Hz ; 1H, H-5), 8.22 (d, J = 8 Hz ; 1H, H-6).

UV (qualitativ, pH 1) : $\lambda_{max}$ = 304 nm.

### Beispiel 4

N-Succinyl-Derivat der Komponente B

50 mg (0,05 mmol) Komponente B werden in 2 ml wasserfreiem Methanol suspendiert. Nach Zugabe von 250 mg (2,5 mmol) Bernsteinsäureanhydrid wird der Ansatz 16 h bei 25 °C gerührt. Das Produkt wird mit 100 ml wasserfreiem Diethylether ausgefällt und abfiltriert. Der Rückstand wird 10 min 5 ml in Ammoniak/Methanol bei 25 °C gerührt, eingeengt und an 5 g Kieselgel (0,063-0,2 mm) chromatographiert. Als Laufmittel wurde ein Gradient Acetonitril : Wasser 4 : 1 → 2 : 1 verwandt.

Ausbeute : 28 mg (51,0 %).

$^1$H-NMR ($D_2$0,250 MHz) : δ = 1.58-1.92 (m ; 12H, β, γ-$CH_2$ Ornithin), 2.14 (s ; 9H, N-Acetyl), 2.52 (s ; 4H, —$CH_2$-Bernsteinsäure), 3.32 (s ; 3H, > N—$CH_3$), 5.95 (d, J = 6 Hz ; 1H, H-1'), 6.22 (d, J = 8 1H, Hz ; H-5), 8.22 (d, J = 8 Hz ; 1H, H-6).

UV (qualitativ, pH 1) : $\lambda_{max}$ = 304 nm.

Beispiel 5

N-L-Valyl-Derivat der Komponente B

50 mg (0,05 mmol) Komponente B und 183,5 mg (0,5 mmol) N-o-Nitrophenylsulfenyl-L-valin-(N-hydroxysuccinimid)-ester werden in 4 ml Wasser : Tetrahydrofuran 1 : 1 gegeben und 16 h bei 25 °C gerührt, wobei mit 0,1 n Triethylamin auf pH 7 eingestellt wurde. Der Ansatz wird am Kugelrohr zur Trockne eingedampft. Der Rückstand wird dreimahl mit 10 ml Diethylether ausgezogen. Die etherische Phase wird verworfen, der Rückstand in einer Lösung aus 4 ml Methanol, 0,4 ml Eisessig und 0,4 ml in Natriumthiosulfatlösung 30 min. bei 25 °C gerührt. Das entstandene Gemisch wird filtriert, das Filtrat mit 10 ml Wasser versetzt und dreimal mit 10 ml Diethylether extrahiert. Die wäßrige Phase wird gefriergetrocknet und an 10 g Kieselgel (0,063-0,2 mm) chromatographiert. Als Laufmittel wurde Acetonitril ; Wasser 8 : 2 verwandt.

Ausbeute : 5,2 mg (9,5 %).

$^1$H-NMR ($D_2O$, 250 MHz) : δ = 0.97 (d breit, J = 6 Hz ; 6H, —$CH_3$ Valin), 1.55-1.89 (m ; 12H, ß, γ-$CH_2$-Ornithin), 2.13 (s ; 9H, N-Acetyl), 3.31 (s ; 3H, > N—$CH_3$), 5.94 (d, J = 6 Hz ; 1H, H-1′), 6.20 (d, J = 8 Hz ; 1H, H-5), 8.20 (d, J = 8 Hz ; 1H, H-6).

UV (qualitativ, pH 1) : $\lambda_{max}$ = 303 nm.

Beispiel 6

N-L-Valyl-Derivat der Komponente A

50 mg (0,052 mmol) Komponente A und 95,4 mg (0,26 mmol) N-o-Nitrophenylsulfenyl-L-valin-(N-hydroxysuccinimid)-ester werden in 5 ml Wasser : Tetrahydrofuran 1 : 1 gegeben. Mit 0,1 n Triethylaminlösung wird auf pH 7 eingestellt und 16 h bei 25 °C gerührt. Die entstandene Lösung wird am Rotationsverdampfer eingeengt (40 °C), der Rückstand wird zweimal mit 10 ml Diethylether ausgezogen. Die etherische Phase wird verworfen, der Rückstand 60 min. bei 25 °C in einer Lösung von 3 ml Methanol, 0,3 ml Eisessig und 0,3 ml 1 n Natriumthiosulfatlösung gerührt. Die entstandene Suspension wird mit 10 ml Wasser verdünnt, filtriert und das Filtrat zweimal mit 10 ml Ether extrahiert. Die wäßrige Phase wird gefriergetrocknet und an 7,5 g Kieselgel (0,063-0,2 mm) chromatographiert. Als Laufmittel wurde Acetonnitril ; Wasser 4 : 1 verwendet.

Ausbeute : 9,5 mg (17,4 %).

$^1$H-NMR ($D_2O$, 250 MHz) : δ = 0,96 (d breit, J = 6 Hz ; 6H, —$CH_3$ Valin), 1,56-1,94 (m ; 12H, β, γ-$CH_2$-Ornithin), 2.13 (s ; 9H, N-Acetyl), 3.29 (s ; 3H, N—$CH_3$), 5.96 (d, J = 6 Hz ; 1H, H-1′), 5.99 (d, J = 8 Hz ; 1H, H-5), 8.49 (d, J = 8 Hz ; 1H, H-6).

UV (qualitativ, pH 1) : $\lambda_{max}$ = 264 nm.

Beispiel 7

N-L-Alanyl-Derivat der Komponente B

50 mg (0,05 mmol) Komponente B und 84,8 mg (0,25 mmol) N-o-Nitrophenylsulfenyl-L-alanyl-(N-hydroxysuccinimid)-ester werden in 5 ml Wasser : Tetrahydrofuran 1 : 1 gelöst. Mit 0,1 n Triethylaminlösung wird auf pH 7 eingestellt. Nach 16 h wird der Ansatz bei 40 °C am Rotationsverdampfer eingeengt, zweimal mit 10 ml Diethylether ausgezogen. Die etherische Phase wird verworfen, der Rückstand 60 Min. bei 25 °C in einer Lösung von 3 ml Methanol, 0,3 ml Eisessig und 0,3 ml Natriumthiosulfat gerührt. Die entstandene Suspension wird mit Wasser verdünnt, dreimal mit 10 ml Diethylester extrahiert und die wäßrige Phase gefriergetrocknet. Das Rohmaterial wird an 7,5 g Kieselgel (0,063-0,2 mm) chromatographiert. Als Laufmittel wurde Acetonitril : Wasser 4 : 1 verwendet.

Ausbeute : 13,9 mg (26,1 %).

$^1$H-NMR ($D_2O$, 250 MHz) : δ = 1,50 (d ; J = 7 Hz ; 3H, CH—$CH_3$-Alanin) ; 1,58-1,92 (m ; 12H, β, γ-$CH_2$ Ornithin) ; 2.14 (s ; 9H, N-Acetyl), 3.32 (s ; 3H, N—$CH_3$) ; 5,94 (d ; J = 6 Hz ; 1H, H-1′), 6,20 (d ; J = 8 Hz ; 1H, H-5) ; 8,20 (d ; J = 8 Hz ; 1H, H-6).

UV (qualitativ, pH 1) : $\lambda_{max}$ = 305 nm.

Beispiel 8

N-Glycyl-Glycyl-Derivat der Komponente B

300 mg (0,3 mmol) Komponente B und 494 mg (1,5 mmol) t-Butoxycarbonylglycylglycyl-(N-hydroxysuccinimid)-ester werden in 60 ml Wasser : Tetrahydrofuran 1 : 1 gelöst. Nach Zugabe von 101 mg (1,2 mmol) Natriumhydrogencarbonat wird 20 h bei 25 °C gerührt. Der Ansatz wird bei 40 °C am Rotationsverdampfer eingeengt und bei 0 °C 30 min mit 10 ml Methylenchlorid/Trifluoressigsäure 1 : 1 gerührt. Die Lösung wird eingeengt, in 10 ml Wasser aufgenommen und mit 1 n Natronlauge auf pH 7 gestellt. Die

Lösung wird an 30 ml Adsorberharz OC 1031 entsalzt und nach Gefriertrocknung an 15 g Kieselgel (0,06-0,2 mm) chromatographiert. Als Laufmittel wurde ein Gradient Acetonitril ; Wasser 9 : 1 → 1 : 1 verwendet.

Ausbeute : 200,2 mg (60,3 %).

$^1$H-NMR ($D_2O$, 250 MHz) : δ = 1.59-1.89 (m ; 12H, β, γ-$CH_2$ Ornithin), 2.12 (s ; 9H, N-Acetyl), 3.31 (s ; 3H, N—$CH_3$), 3.65 (m ; 6H, δ-$CH_2$ Ornithin), 3.82 (s ; 2H, σ-$CH_2$ Glycin), 4.01 (s ; 2H, σ-$CH_2$ Glycin), 5.94 (d, J = 6 Hz ; 1H, H-1'), 6.20 (d, J = 8 Hz ; 1H, H-5) ; 8.21 (d, J = 8 Hz ; 1H, H-6).

UV (qualitativ, pH 1) : $\lambda_{max}$ = 304 nm.

Beispiel 9

N-Glycyl-Glycyl-Derivat der Komponente A

Ansatzgröße und Durchführung wie Beispiel 8.

Ausbeute : 222,8 mg (66,4 %).

$^1$H-NMR ($D_2O$, 250 MHz) : δ = 1.55-1.89 (m ; 12H β, γ-$CH_3$ Ornithin), 2.12 (s ; 9H, N-Acetyl), 3.28 (s ; 3H, N—$CH_3$), 3.78 (s ; 2H, σ-$CH_2$ Glycin), 4.01 (s ; 2H, σ-$CH_2$ Glycin), 5.94 (d, J = 6 Hz ; 1H, H-1'), 5.98 (d, J = 8 Hz ; 1H, H-5), 8.48 (d, J = 8 Hz ; 1H, H-6).

UV (qualitativ, pH 1) : $\lambda_{max}$ = 266 nm.

**Ansprüche**

1. Verbindungen der Formel I

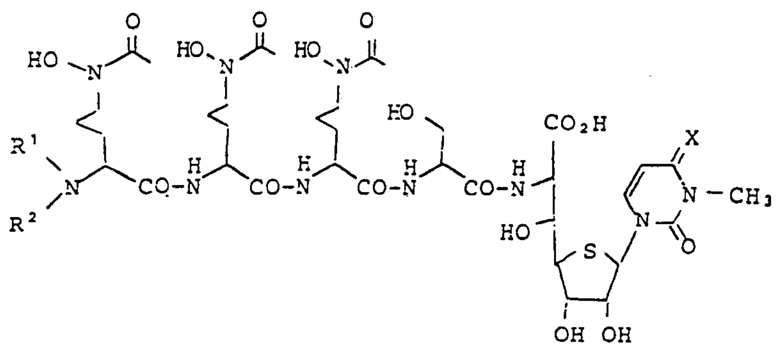

(I)

in der

X = O oder N—CO—$CH_2$ bedeutet und

$R_1$ eine gegebenenfalls substituierte Alkylgruppe oder die Acylgruppe —CO—$R_3$,

$R_2$ Wasserstoff oder $R_1$ und

$R_3$ eine gegebenenfalls substituierte Alkyl-, Aryl- oder Aminogruppe

darstellt.

2. Verbindungen nach Anspruch 1, dadurch gekennzeichnet, daß $R_1$ eine Alkylgruppe mit bis zu 6 C-Atomen darstellt.

3. Verbindungen nach Anspruch 1, dadurch gekennzeichnet, daß $R_3$ eine Alkylgruppe mit bis zu 6C-Atomen darstellt.

4. Verbindungen nach Anspruch 1, dadurch gekennzeichnet, daß $R^1$ den Acylrest einer Aminosäure oder eines Di- bis Pentapeptids und $R^2$ = H bedeutet.

5. Verfahren zur Herstellung der Verbindungen gemäß Anspruch 1, dadurch gekennzeichnet, daß man

a) zur Herstellung der Verbindungen mit $R^1 = R^2$ = H unter submersen, aeroben Bedingungen Streptomyces spec. WS 116 (DSM 1692) oder von diesem abstammende Mutanten und Varianten in einem assimilierbaren Kohlenstoff, Stickstoff sowie Mineralsalze, insbesondere Eisensalze enthaltenden Nährmedium bei einer Temperatur von 15 bis 35 °C züchtet und die gebildeten Verbindungen aus der Fermentationsflüssigkeit isoliert und auftrennt und

b) die so erhaltenen Verbindungen in an sich bekannter Weise an der freien Aminogruppe derivatisiert.

6. Verfahren nach Anspruch 5, dadurch gekennzeichnet, daß man in Verfahrensschnitt a) zur Isolierung das rohe Produkt über einen Kationenaustauscher in der Fermentations-$Fe^{3+}$-Form chromatographiert, inaktive Begleitstoffe mit einem Puffer hoher Ionenstärke eluiert und das Produkt mit dem gleichen Puffer und Zusatz eines Eisen-Komplexbildners eluiert.

7. Verfahren nach den Ansprüchen 5 und 6, dadurch gekennzeichnet, daß man das nach Anspruch 6 erhaltene Produkt über eine CM-Cellulose-Säule in der $H^+$-Form chromatographiert und mit Wasser fraktioniert eluiert.

8. Verfahren nach den Ansprüchen 5-7, dadurch gekennzeichnet, daß in einer Nährlösung fermentiert wird, die als Hauptkohlenstoffquelle Citronensäure, mehrere komplexe Stickstoffquellen

(Hefeextrakt, Sojabohnenmehl, Maisquellwasser) und die Aminosäuren L-Ornithin und L-Serin erhält.

9. Arzneimittel mit einem Gehalt an der Verbindung gemäß einem der Ansprüche 1-4.

10. Verbindungen gemäß einem der Ansprüche 1-4 Zur Verwendung bei der Bekämpfung bakterieller Infektionen.

**Claims**

1. Compounds of the formula I

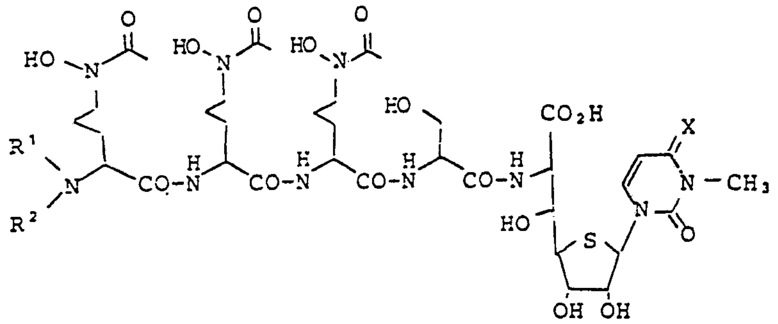

(I)

in which

X = O or denotes N—CO—$NH_2$,

$R_1$ represents an optionally substituted alkyl group or the acyl group —CO—$R_3$,

$R_2$ represents hydrogen or $R_1$ and

$R_3$ represents an optionally substituted alkyl, aryl or amino group.

2. Compounds according to Claim 1, characterised in that $R_1$ represents an alkyl group having up to 6 C atoms.

3. Compounds according to Claim 1, characterised in that $R_3$ represents an alkyl group having up to 6 C atoms.

4. Compounds according to Claim 1, characterised in that $R^1$ denotes the acyl radical of an amino acid or of a dipeptide to pentapeptide and $R^2$ denotes H.

5. Process for the preparation of the compounds according to Claim 1, characterised in that

a) for the preparation of the compounds with $R^1 = R^2 = H$, Streptomyces spec. WS 116 (DSM 1692), or mutants and variants derived therefrom, are grown under submerse, aerobic conditions in a nutrient medium containing assimilable carbon, nitrogen and mineral salts, in particular iron salts, at a temperature of 15 to 35 °C, and the compounds formed are isolated from the fermentation liquid and are separated, and

b) the compounds thus obtained are derivatised at the free amino group, in a manner which is in itself known.

6. Process according to Claim 5, characterised in that in process step a) the isolation is carried out by chromatographing the crude product over a cation exchanger in the fermentation $Fe^{3+}$ form, eluting inactive accompanying substances with a buffer of high ionic strength, and eluting the product with the same buffer, with the addition of an iron complex-former.

7. Process according to Claims 5 and 6, characterised in that the product obtained according to Claim 6 is chromatographed over a CM cellulose column in the $H^+$ form and fractionally eluted with water.

8. Process according to Claims 5-7, characterised in that the fermentation is carried out in a nutrient solution which contains citric acid as the main carbon source, several complex nitrogen sources (yeast extract, soya bean meal, corn steep liquor), and the amino acids L-ornithine and L-serine.

9. Medicaments containing the compound according to one of the Claims 1-4.

10. Compounds according to one of the Claims 1-4 for use in combating bacterial infections.

**Revendications**

1. Composés de formule I

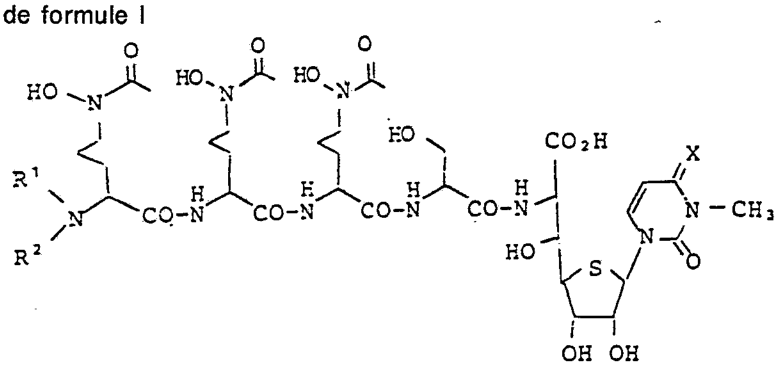

(I)

dans laquelle

X = O ou N—CO—$NH_2$ et

$R_1$ est un groupe alkyle éventuellement substitué ou le groupe acyle —CO—$R_3$,

$R_2$ est l'hydrogène ou $R_1$ et

$R_3$ est un groupe alkyle, aryle ou amino éventuellement substitué.

2. Composés suivant la revendication 1, caractérisés en ce que $R_1$ est un groupe alkyle ayant jusqu'à 6 atomes de carbone.

3. Composés suivant la revendication 1, caractérisés en ce que $R_3$ est un groupe alkyle ayant jusqu'à 6 atomes de carbone.

4. Composés suivant la revendication 1, caractérisés en ce que $R^1$ est le reste acyle d'une aminoacide ou d'un di- à pentapeptide et $R^2$ = H.

5. Procédé de production des composés suivant la revendication 1, caractérisé en ce que

a) pour l'obtention des composés dans lesquels $R^1 = R^2 = H$, on cultive dans des conditions aérobies en immersion Streptomyces spec. WS 116 (DSM 1692) ou des mutants et variants qui en proviennent, dans un milieu nutritif contenant du carbone assimilable, de l'azote, ainsi que des sels minéraux, notamment des sels de fer, à une température de 15 à 35 °C, et on isole du liquide de fermentation les composés formés et on les sépare et

b) on forme d'une manière connue des dérivés des composés ainsi obtenus sur le groupe amino libre.

6. Procédé suivant la revendication 5, caractérisé en ce que pour effectuer l'isolement dans l'étape a) du procédé, on chromatographie ce produit brut sur un échangeur cationique sous la forme $Fe^{3+}$ de fermentation, on élue les impuretés inactives avec un tampon de grande force ionique et on élue le produit avec le même tampon avec addition d'un agent de complexation du fer.

7. Procédé suivant les revendications 5 et 6, caractérisé en ce qu'on chromatographie le produit obtenu selon la revendication 6 sur une colonne de CM-cellulose sous la forme $H^+$ et on procède à une élution fractionnée avec de l'eau.

8. Procédé suivant les revendications 5-7, caractérisé en ce qu'on conduit la fermentation dans une solution nutritive qui contient comme source principale de carbone de l'acide citrique, plusieurs sources complexes d'azote (extrait de levure, farine de soja, extrait soluble de maïs) et les aminoacides L-ornithine et L-sérine.

9. Médicament renfermant une teneur en le composé suivant l'une des revendications 1-4.

10. Composés suivant l'une des revendications 1-4, destinés à être utilisés pour combattre des infections bactériennes.

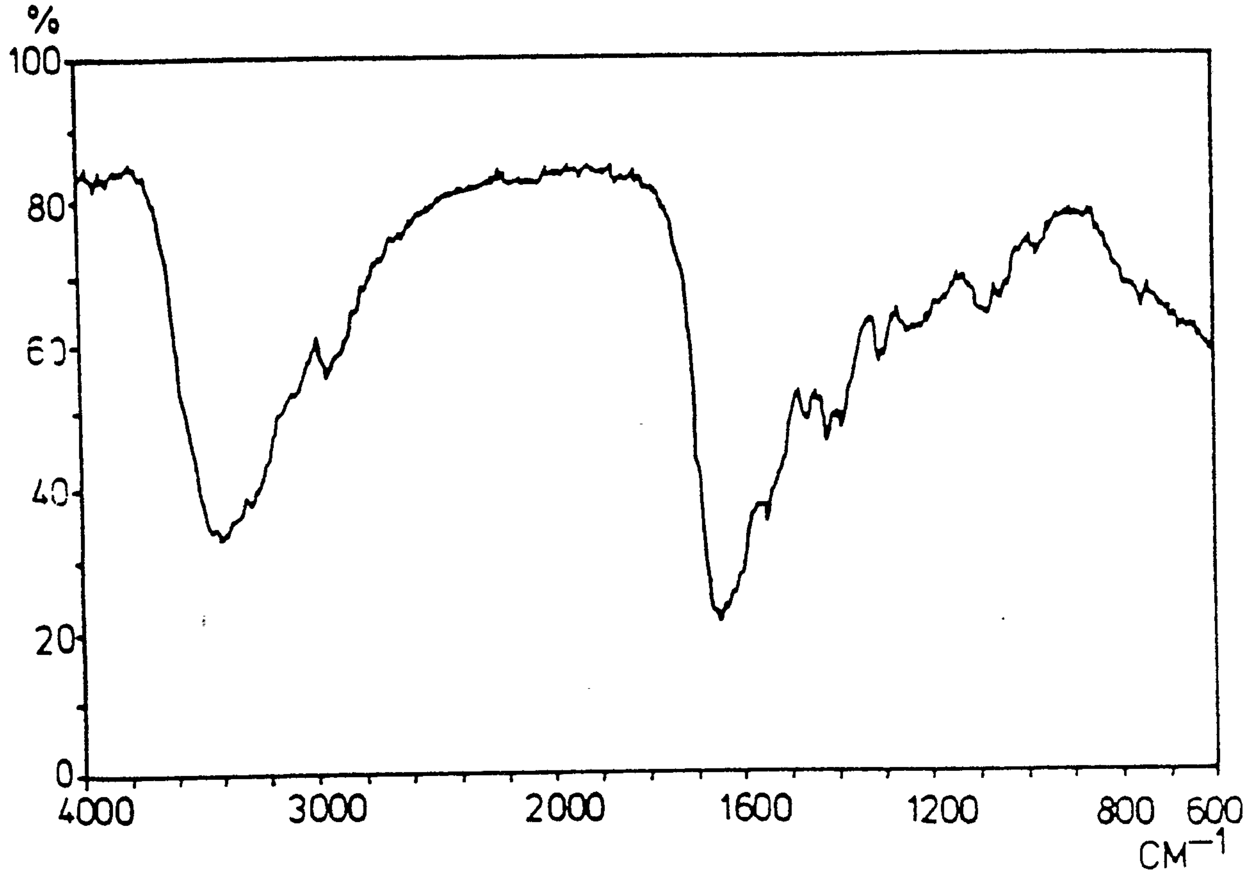


FIG. 1

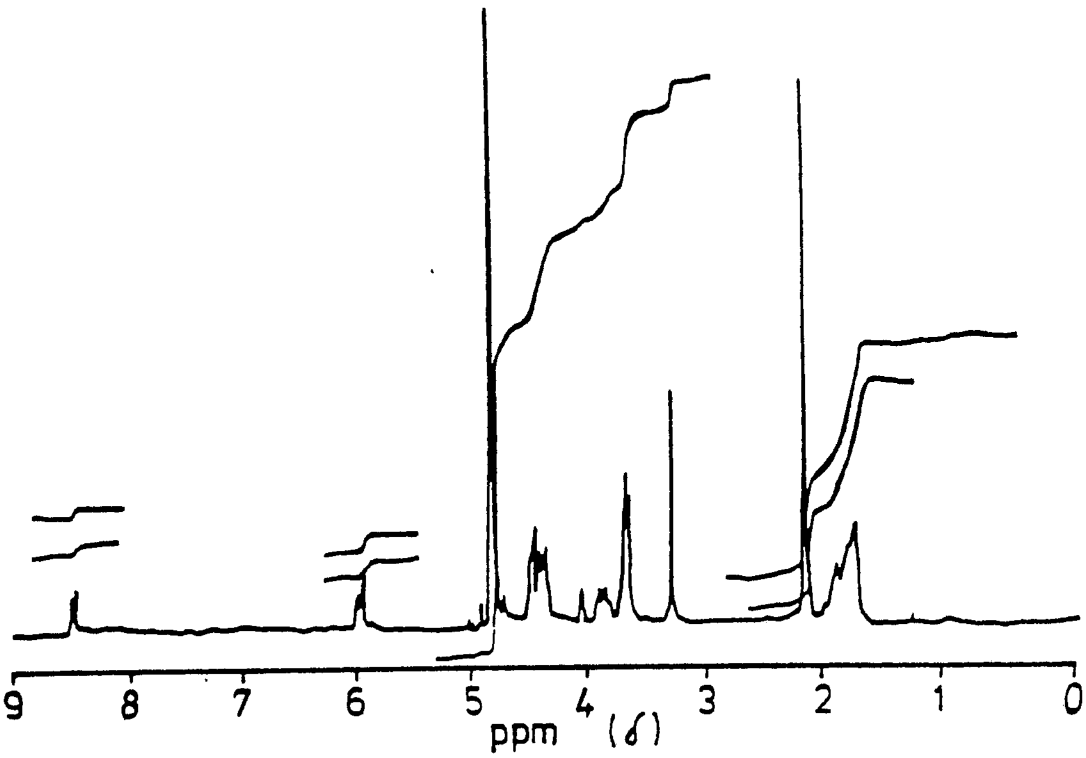


FIG. 2

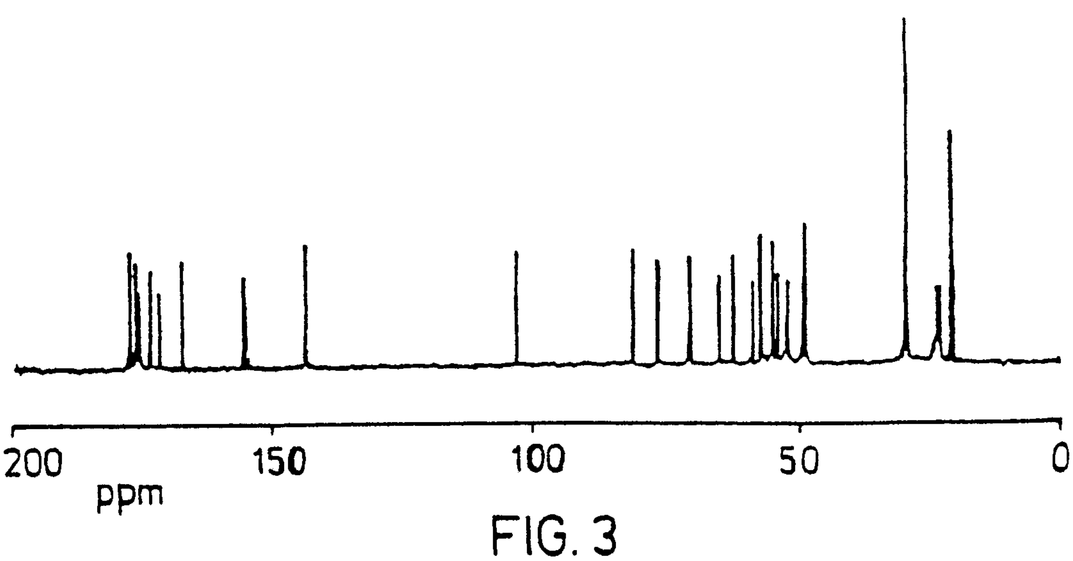


FIG. 3

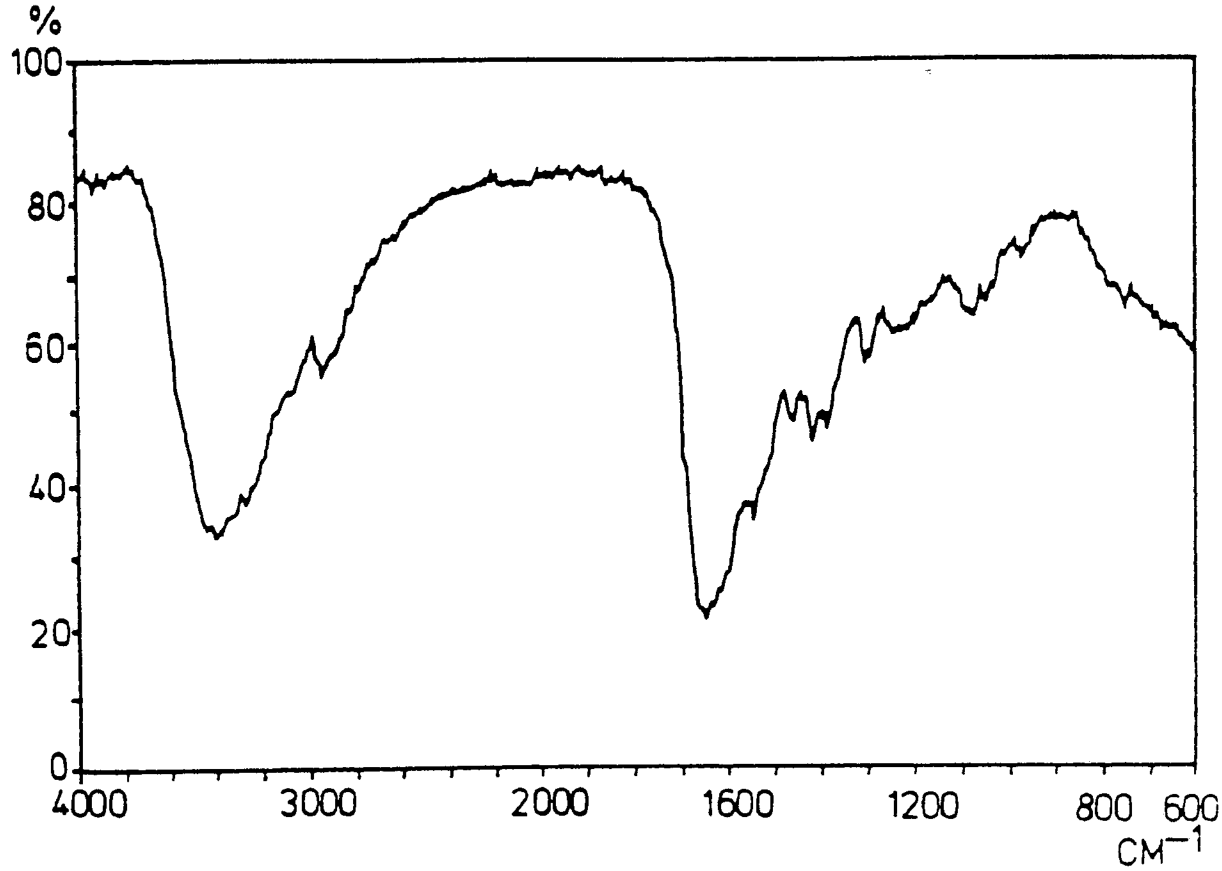


FIG. 4

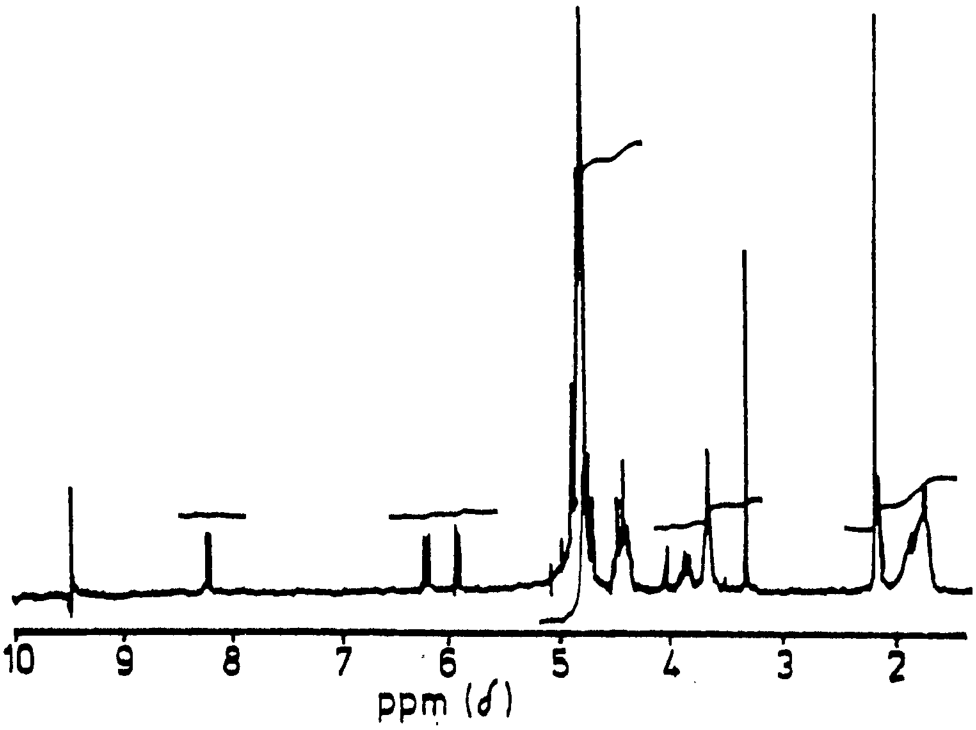


FIG. 5

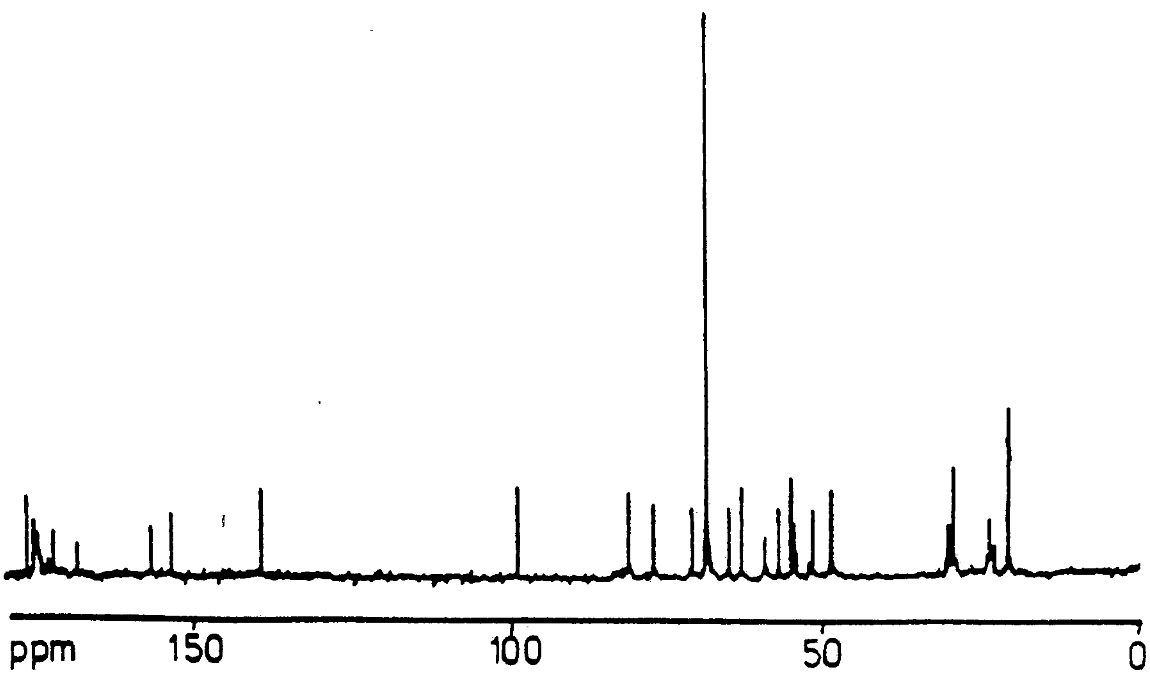


FIG. 6